# EUROPÄISCHE PATENTSCHRIFT

(11) **EP 4 285 871 B1**
(45) Veröffentlichungstag und Bekanntmachung des Hinweises auf die Patenterteilung: **01.04.2026**
(21) Anmeldenummer: 23205304.1
(22) Anmeldetag: 20.09.2017
(51) Int. Cl.: A61F 2/58, A61F 2/68, A61F 2/70, A61F 2/76, A61F 2/80

(54) **FREMDKRAFTBETÄTIGBARE PROTHESE**
PROSTHESIS THAT CAN BE ACTUATED BY EXTERNAL FORCE
PROTHÈSE POUVANT ÊTRE ACTIONNÉE PAR UNE FORCE EXTÉRIEURE

(30) Priorität: 26.09.2016 CH 12522016
(43) Veröffentlichungstag der Anmeldung: 06.12.2023
(62) Teilanmeldung aus: 17781019.9
(73) Patentinhaber: Appsocial.org Stiftung, 8032 Zürich (CH)
(72) Erfinder: SCHOLLENBERGER, Fabian, 8127 Forch (CH); ELSPASS, Wilfried J., 8542 Wiesendangen (CH); LENGACHER, Lukas, 8305 Dietlikon (CH); MOSER, Tobias, 8637 Laupen ZH (CH)
(74) Vertreter: Keller Schneider Patent- und Markenanwälte AG

(56) Entgegenhaltungen:
- WO-A1-98/48740
- CN-A- 101 849 865
- US-A- 5 413 611
- US-A1- 2010 198 362
- US-A1- 2010 274 365
- US-A1- 2014 277 588
- NAKAMURA Y ET AL: "CLINICAL APPLICATION OF AN ELECTRICAL POWERED UPPER LIMB PROSTHESISWITH SENSORY FEEDBACK SYSTEM TO A PATIENT WITH ABOVE ELBOW AMPUTATION AND BRACHIAL PLEXUS INJURY", BIOMECHANICS, REHABILITATION, ELECTRICAL PHENOMENA, BIOMATERIALS. SAN DIEGO, OCT. 28 - 31, 1993; [PROCEEDINGS OF THE ANNUAL INTERNATIONAL CONFERENCE OF THE ENGINEERING IN MEDICINE AND BIOLOGY SOCIETY], NEW YORK, IEEE, US, vol. 15, PART 03, 28 October 1993 (1993-10-28), XP000452860

## Beschreibung

### Technisches Gebiet

Die vorliegende Erfindung beschreibt eine fremdkraftbetätigbare Prothese, umfassend einen Prothesenschaft und eine Exoprothese mit mindestens einem mittels einer Prothesensteuerungsvorrichtung betätigbaren Prothesenmotor und einem Gelenk, wobei die Prothesensteuerungsvorrichtung ein Gehäuse, einen darin angeordneten Mikrokontroller und Anzeigemittel umfasst, sodass ein Greifprozess durch den von der Prothesensteuerungsvorrichtung steuerbaren Prothesenmotor nachbildbar ist und Parameter des Greifprozesses von den Anzeigemitteln darstellbar sind, wobei auf die Aufnahme und Auswertung von Elektromyografie-Signalen verzichtet wird.

### Stand der Technik

Heute sind bevorzugt Prothesen, umfassend einen Prothesenschaft mit einer Exoprothese in Form einer Hand- oder Fussprothese gefragt, welche fremdkraftbetätigt sind. Dabei wird eine Prothesensteuerungsvorrichtung mit der Exoprothese verbunden, mittels welcher mindestens ein Prothesenmotor angesteuert wird, sodass mindestens ein Gelenk an der Exoprothese bewegt werden kann. Im Falle einer Handprothese wird so die Bewegung mindestens eines Fingergelenkes nachgebildet, womit eine Greifbewegung möglich ist.

Da passende elektronische Bauteile erhältlich und erschwinglich sind und möglichst realistische Nachbildungen natürlicher Hand- und Fussbewegungen anvisiert werden, werden Prothesen verbreitet mit Myoelektrik zur Verwendung von Elektromyografie (EMG) ausgerüstet. Durch EMG kann die elektrische Muskelaktivität ermittelt werden, woraus Steuersignale für die Prothesensteuerungsvorrichtung generiert werden können. Mittels Oberflächenelektroden auf der Haut im Bereich des Extremitätenstumpfes eines Prothesenträgers wird der mindestens eine Prothesenmotor der Exoprothese gesteuert. Die abgreifbaren Muskelsignale im Verlauf des Prothesenschafts werden mit der Prothesensteuerungsvorrichtung mittels Sensoren erfasst und in Bewegungen der Exoprothese umgesetzt.

Neben einem gesteigerten technischen Aufwand für derartige myoelektrisch gesteuerte fremdkraftbetätigte Prothesen ist es notwendig, dass der Prothesenträger die Ansteuerung mittels myoelektrischer Signale trainiert. Die Nutzung einer Prothese mit EMG ist daher generell nur mit einem Lernprozess zur Ansteuerung der Prothese möglich, welcher oftmals langwierig ist. Die Algorithmen zur Erkennung der Benutzereingabe sind schwierig einstellbar und oft nicht reproduzierbar, sodass die Ansteuerung oft Probleme bereitet, wenn der Prothesenträger beispielsweise schwitzt und/oder sich die Oberflächenelektroden verschieben. Zusätzlich kann bei längerem Tragen solcher Prothesen das EMG-Signal, durch das Ermüden einzelner Muskelpartien, markant absinken.

Um die bekannten Nachteile der Prothesen auf Basis von EMG-Signalen zu beseitigen, werden immer aufwändigere Lösungsvorschläge gemacht, was die Komplexität dieser Art von Prothesen weiter erhöht. Oftmals werden weitere Sensoren verwendet, um beispielsweise eine Greifbewegung noch genauer zu detektieren. Entsprechend wird die Prothesensteuerungsvorrichtung noch aufwändiger und die Prothesen werden noch teurer, was beispielsweise den Einsatz der Prothesen in der dritten Welt erschwert.

Beispielsweise ist in der WO2013038187 ein Verfahren zur Steuerung einer EMG-Prothese beschrieben, welches dem Prothesenträger erlaubt Prothesenbewegungen zu trainieren, selbst wenn die Prothese nicht einsatzbereit ist. Mit den aufgenommenen EMG-Signalen können wahlweise die Exoprothese bewegt oder Bewegungen in einem Computer simuliert werden, wobei durch ein Prothesenträger durch letzteres reale Bewegungen trainieren kann. Der Prothesenträger kann vordefinierte Programme, beispielsweise für unterschiedliche Greifvorgänge durch die richtigen Muskelsignale via Computer starten und somit durch die Exoprothese ausführen. Neben einer Vielzahl von EMG-Sensoren, nach Programmierung der vordefinierten Programme und durch Nutzung einer entsprechenden Prothesensteuerungsvorrichtung, können Greifprogramme computerunterstützt ablaufen. Dies ist aber mit entsprechend weiter erhöhtem technischen Aufwand verbunden. Ausserdem kann ein Prothesenträger nur die gespeicherten Programme, angesteuert durch seine EMG-Signale von der Prothese durchführen lassen, was wenig flexibel ist. Die Sensorik, Aktorik und Elektronik für die Regelung der beschriebenen Prothesen ist kostenintensiv und sehr komplex.

Um vereinfachte und vor allem erschwingliche Prothesen zu schaffen, welche reproduzierbare Bewegungen bzw. Motoransteuerungen zeigen, wird versucht die Prothesensteuerungsvorrichtung und den Prothesenmotor noch einfacher zu gestalten und auf EMG-Sensoren und zusätzliche Computerunterstützung zu verzichten. Verzichtet der Fachmann auf EMG-Sensoren, ist nicht klar, wie reproduzierbare einfach durchführbare Greifbewegungen durchgeführt werden können. Vor allem zerbrechliche Objekte können mit dem Fachmann bekannten rudimentären Prothesen meist ohne Sensorunterstützung nicht mehr gegriffen werden.

Aus US 2010/198362, die als nächstliegender Stand der Technik angesehen werden kann, und US 2010/274365 kann eine fremdkraftbetätigbare Prothese mit einem Prothesenmotor und einer Prothesensteuerungsvorrichtung entnommen werden, womit ein Greifprozess durch den von der Prothesensteuerungsvorrichtung steuerbaren Prothesenmotor nachbildbar ist und auf die Aufnahme und Auswertung von Elektromyografie-Signalen verzichtet werden kann.

### Darstellung der Erfindung

Die vorliegende Erfindung hat sich zur Aufgabe gestellt eine einfache fremdkraftbetätigbare und kostengünstige Prothese zu schaffen, welche ohne aufwändiges Training bedienbar ist und mit wenigen Bauteilen auskommt. Mit dieser Prothese ist ein reproduzierbares zerstörungsfreies und sicheres fremdkraftgesteuertes Greifen unterschiedlich harter Objekte erreichbar, wobei auf die Auswertung von Elektromyografie-Signalen und Griffkraftsensoren verzichtet wird.

Diese Aufgabe wird dadurch gelöst, dass die Prothesensteuerungsvorrichtung der Prothese durch einen Prothesentäger vor und während der Greifbewegung von aussen manipulierbar ist, wobei die Griffkraft und die Griffdauer durch den Prothesenträger einfach und verlässlich eingestellt werden und auch während der Greifbewegung in Echtzeit durch den Prothesenträger regulierbar ist. Dabei werden unter anderem die eingestellte Griffkraft und Griffdauer dem Prothesenträger optisch, taktil und/oder akustisch übermittelt werden.

### Kurze Beschreibung der Zeichnungen

Ein bevorzugtes Ausführungsbeispiel des Erfindungsgegenstandes wird nachstehend im Zusammenhang mit den anliegenden Zeichnungen beschrieben.
- Figur 1a: zeigt eine Aufsicht auf eine Prothese mit Prothesensteuerungsvorrichtung an einem Armband, um den Prothesenschaft gelegt mit Vergrösserung der Anzeigemittel in Form eines Bildschirms, während
- Figur 1b: eine Aufsicht auf eine Prothese mit Prothesensteuerungsvorrichtung in Form eines Sportarmbandes am Prothesenschaft befestigt zeigt.
- Figur 2: zeigt eine perspektivische Ansicht einer Handprothese nach Abkopplung vom Prothesenschaft mit darin integrierter Prothesensteuerungsvorrichtung.
- Figur 3: zeigt eine schematische Ansicht eines Touchdisplays als Eingabe- und Anzeigemittel, welches am Prothesenschaft oder der Handprothese befestigt werden kann.
- Figur 4: zeigt eine Explosionsdarstellung einer Prothesensteuerungsvorrichtung allein, während
- Figur 5: eine perspektivische Darstellung der Prothesensteuerungsvorrichtung, angeordnet zwischen Prothesenschaft und Handprothese, zeigt.

### Beschreibung

Hier wird eine fremdkraftgesteuerte Prothese 1, umfassend einen Prothesenschaft 2, eine Kupplung 3, eine Exoprothese 4 und eine Prothesensteuerungsvorrichtung 5 am Beispiel einer Handprothese gezeigt und erläutert. Entsprechend ist hier der Prothesenschaft 2 über einen Extremitätenstumpf 0 in Form eines Unterarmstumpfes 0, mit gestrichelten Linien angedeutet, gestülpt und lösbar befestigt. Mittels der Kupplung 3 ist die Handprothese 4 mit dem Prothesenschaft 2 verbunden. An der Handprothese 4 befindet sich mindestens ein motorgetriebenes Gelenk, mittels welchem das Greifen eines Objektes realisierbar ist. Ein Prothesenmotor ist hier nicht dargestellt, wird aber derart ausgeführt, dass er von der Prothesensteuerungsvorrichtung 5 steuerbar ist, wodurch ein Greifprozess gebildet wird.

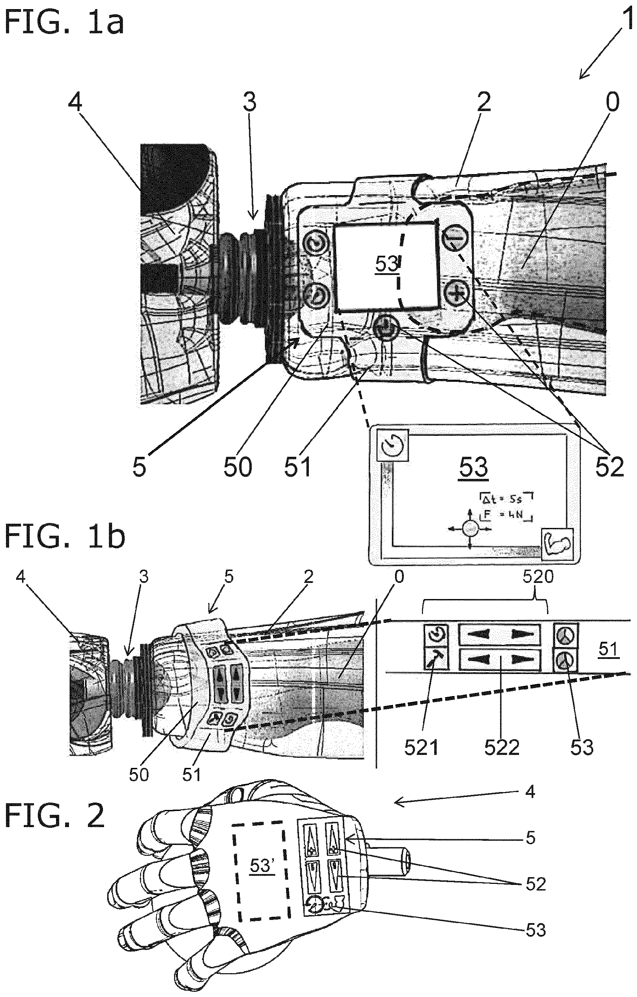

Die Prothesensteuerungsvorrichtung 5 weist ein Gehäuse 50 auf. Das Gehäuse 50 ist an einem Armband 51 befestigt, welches um den Prothesenschaft 2 gelegt ist, sodass die Prothesensteuerungsvorrichtung 5 in Form einer Armbanduhr (Fig. 1a) oder eines Sportarmbandes (Fig. 1b) ausbildbar ist.

Zur erleichterten Bedienung ist die Prothesensteuerungsvorrichtung 5 an der äusseren Oberfläche des Prothesenschafts 2 befestigt und damit beabstandet von der Haut des Prothesenträgers. Dies ist ohne weiteres möglich, da von der Prothesensteuerungsvorrichtung 5 keine myoelektrischen Signale aufgenommen werden.

Die Prothesensteuerungsvorrichtung 5 bzw. das Gehäuse 50 weisen Eingabemittel 52 und Anzeigemittel 53 auf. Die Eingabemittel 52 können neben klassischen Druckschaltern, Tastern, Drehpotentiometern, Knöpfen oder Schaltern, auch aus auf einer Bedienoberfläche 520 angeordneten virtuellen Eingabeknöpfen bestehen. Die Bedienoberfläche 520 der Prothesensteuerungsvorrichtung 5 ist von einer Aussenfläche der Exoprothese 4, des Prothesenschafts 2 oder der Kupplung 3 wegragend angeordnet und damit leicht zugänglich. Alle Eingabemittel 52 sind mechanisch von einem Prothesenträger manuell, also mit einem Finger bedienbar. Die Bedienung erfolgt bevorzugt mit mindestens einem Finger der intakten Hand, könnte aber auch durch ein in der intakten Hand gehaltenes Hilfsmittel bedient werden.

In Figur 1a ist ein Bildschirm als Anzeigemittel 53 mit einer Anzeigefläche ausgeführt, während die Eingabemittel 52 Tasten neben dem Bildschirm 53 sind.

Die Prothesensteuerungsvorrichtung 5 ermöglicht die Anzeige der aktuellen Griffkraft und der Griffdauer auf dem Bildschirm 53, sodass ein Prothesenträger einfach ablesen kann, mit welchen Parametern die nächste Greifbewegung ablaufen wird. Durch die Anzeigemittel 53 kann optisch verfolgt werden, wie die Greifbewegung ablaufen wird oder abläuft.

Die Prothesensteuerungsvorrichtung 5 am Prothesenschaft 2, wie in Figur 1b dargestellt, weist ein Gehäuse 50 mit einem Armband 51 in Form eines Sportarmbandes oder Fitnessarmbandes auf. Über Taster 52, welche auch als kapazitive oder resistive Schalter 52 bzw. Druckelemente ausgebildet werden können, kann der Prothesenträger Parameter in die Prothesensteuerungsvorrichtung 5 eingeben. Als Eingabemittel 52 sind Auswahltasten 521 vorgesehen, mit welchen ein Modus oder ein einzustellender Parameter einstellbar ist. Um die Grösse des gewählten Parameters, beispielsweise der Griffkraft einzustellen, sind Grössenwahltasten 522 vorgesehen. Die hier verwendeten Anzeigemittel 53 können im einfachen Fall LEDs sein. Dabei kann die Anzahl leuchtender LEDs oder die Farbe der leuchtenden LEDs die eingestellten Parameter, wie Griffkraft oder Griffdauer optisch anzeigen.

In einer weiteren Abwandlung ist die Prothesensteuerungsvorrichtung 5 in die Exoprothese 4 intregriert, sodass ein Prothesenträger die Parametereinstellung und das Ablesen der Werte direkt an der Handprothese 4 vornehmen kann. Das Gehäuse 50 ist entsprechend in die Exoprothese 4 integriert bzw. ein Teil der Handprothese 4 bildet das Gehäuse 50 der Prothesensteuerungsvorrichtung 5. Die Eingabemittel 52 und Anzeigemittel 53 und gegebenenfalls optionale Anzeigemittel 53' befinden sich direkt auf der Exoprothese 4.

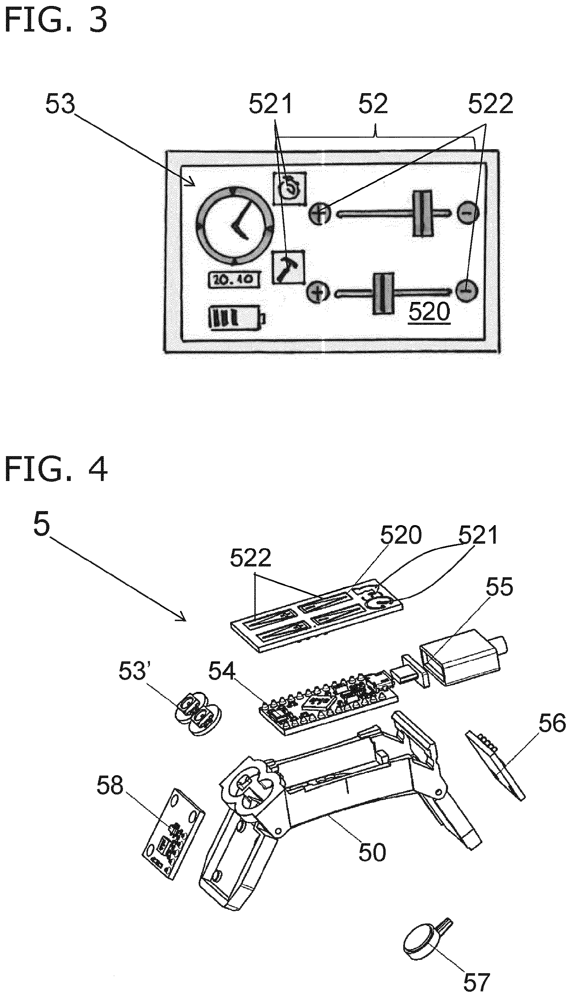

Das Anzeigemittel 53 kann auch als berührungsempfindlicher Bildschirm ausgestaltet sein, wie in Figur 3 dargestellt. Bevorzugt wird eine Betriebssoftware (firmware) oder Applikation (App) auf der Prothesensteuerungsvorrichtung 5 betrieben, welche die Anzeigemittel 53 und die Eingabemittel 52 steuert und darstellt. Als Eingabemittel 52 ist eine Bedienoberfläche 520 auf dem berührungsempfindlichen Bildschirm vorgesehen. Die Bedienoberfläche 520 umfasst hier Eingabemittel 52 in Form von virtuellen Auswahltasten 521 und virtuellen Grössenwahltasten 522 in Form von Schiebereglern. Bei Einsatz eines berührungsempfindlicher Bildschirms und einer App sind vielfältige Ergebnisse erreichbar, wobei Anzeigemittel 53 und Eingabemittel 52 auf dem berührungsempfindlichen Bildschirm vorgesehen sind.

Da die Prothesensteuerungsvorrichtung 5 bevorzugt einen Einplatinencomputer, beispielsweise ein Arduinoboard oder einen Raspberry Pi, umfasst, kann die Prothesensteuerungsvorrichtung 5 neben der Steuerung der Exoprothese auch zum Betrieb weiterer Apps verwendet werden, wie dies von Smartphones und Smartwatches bekannt ist. Damit kann ein Prothesenträger zusätzlich zur Steuerung der fremdkraftbetätigbaren Prothese Apps zur Musik- oder Videowiedergabe, Spieleanwendungen, Nachrichtenübermittlungen ausführen oder sogar telefonieren. Natürlich ist ein Mehrprozessbetrieb dabei wichtig, wobei die Prothesensteuerung immer Vorrang vor den anderen Funktionen hat.

Eine Prothesensteuerungsvorrichtung 5 ist im Detail in Figur 4 gezeigt. Im Gehäuse 50 lagert ein Mikrokontroller 54 als Herzstück der Prothesensteuerungsvorrichtung 5, verbunden mit einer Spannungsversorgung 55. Die Spannungsversorgung 55 ist in Form eines Akkus oder einer oder mehrerer Knopfbatterien gebildet. Eine Bedienoberfläche 520 stellt das Eingabemittel 52 dar und LEDs 53' die Anzeigemittel 53. Über den Mikrokontroller 54 nach Eingabe von Parametern werden die aktuell eingestellten Parameter optisch mittels der LEDs 53' dargestellt. Ebenfalls mit dem Mikrokontroller 54 verbunden ist ein haptischer Motorentreiber 56 und ein Vibrationsmotor 57. Ausgelöst durch den Mikrokontroller 54 führt der Vibrationsmotor 57 Vibrationen aus, die der Prothesenträger fühlen kann, wenn die Prothesensteuerungsvorrichtung 5 am Prothesenschaft 2 oder direkt an der Handprothese 4 angeordnet ist. Durch den haptischen Motorentreiber 56 in Verbindung mit dem Vibrationsmotor 57 wird dem Prothesenträger das Erkennen des Griffkontaktes der Handprothese 4 durch Vibrationen ermöglicht. Das Ziel ist es die eingestellten Parameter dem Prothesenträger auch taktil mitzuteilen, wobei eine Vibration erfolgen kann, sobald die Griffbewegung mit der eingestellten Griffkraft erfolgt ist bzw. wenn ein Objekt gegriffen wurde.

Die Bedienoberfläche 520 ist hier als berührungsempfindliches PCB-Bauteil (gedruckte Schaltung auf einer Leiterplatte, printed circuit board) ausgebildet, wobei auf berührungsempfindlichen Flächen (touch-pads) die Eingabe erfolgt. Auf den Grössenwahltasten 522, als pfeilförmig gestaltete Flächen dargestellt, können gewählte Parameter vergrössert bzw. verkleinert werden. Mittels Auswahltasten 521 können die einzustellenden Parameter variiert werden. Die auf der Bedienoberfläche 520 eingegebenen Werte werden an den Mikrokontroller zur weiteren Verarbeitung weitergeleitet.

Optional kann in die Prothesensteuerungsvorrichtung 5 ein Beschleunigungssensor 58 integriert sein, welcher ebenfalls an den Mikrokontroller 54 angeschlossen ist. Durch den Beschleunigungssensor 58 können die Bewegungen und Beschleunigungen der am Prothesenschaft 2 und/oder der Exoprothese 4 mitgeführten Prothesensteuerungsvorrichtung 5 registriert werden.

Die Prothesensteuerungsvorrichtung 5 steuert mittels Mikrokontroller 54, auf welchem eine Betriebssoftware läuft, einen Greifprozess. Durch Eingabemittel 52 werden Parameter von der Bedienoberfläche 520 an den Mikrokontroller 54 übermittelt, vom Mikrokontroller 54 verarbeitet und mit den Anzeigemitteln 53 angezeigt. Gesteuert durch den Mikrokontroller 54 wird die Prothesenmotorbewegung, wie eingestellt gesteuert. Auch während des Greifprozesses ist eine Nachjustierung der Parameter mittels Eingabemitteln 52 durch den Prothesenträger in Echtzeit möglich.

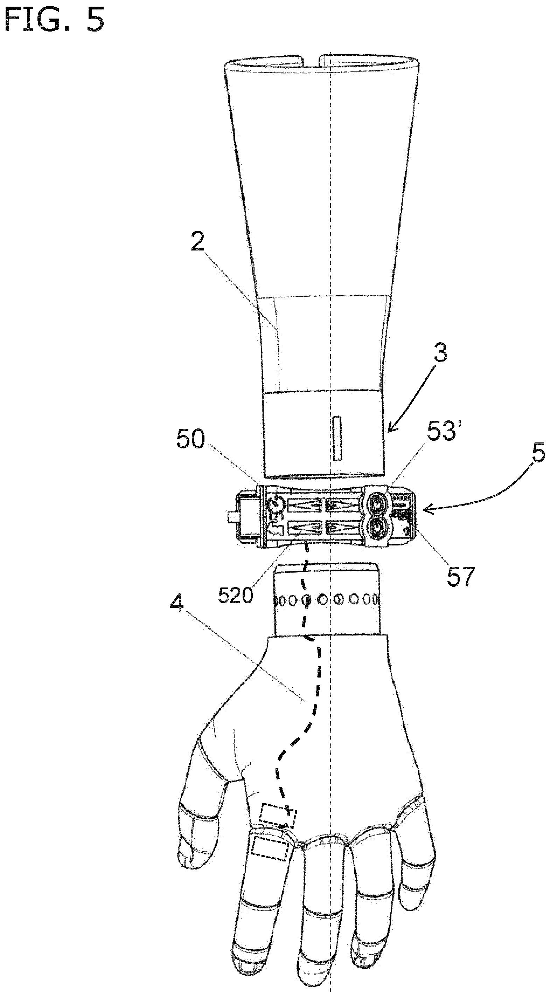

Die Spannungsversorgung 55 ist im Prothesenschaft 2, an der Exoprothese 4 angeordnet, könnte aber auch anderweitig am Körper des Prothesenträgers, beispielsweise in einer Hosentasche gelagert werden. Wie in Figur 5 gezeigt, kann die Prothesensteuerungsvorrichtung 5 die Kupplung 3 zwischen Prothesenschaft 2 und Exoprothese 4 mindestens teilweise verdeckend angeordnet sein. Skizziert ist hier eine Verbindung von der Prothesensteuerungsvorrichtung 5 zu mindestens einem Fingergelenk der Handprothese 4, welches durch die Prothesensteuerungsvorrichtung 5 bewegbar ist. Ein Prothesenmotor, der die Griffbewegung durchführen kann, ist gestrichelt angedeutet. Die Verbindung zwischen Prothesensteuerungsvorrichtung 5 und Handprothese 4 bzw. dem mindestens einen Prothesenmotor kann durch ein Kabel oder drahtlos, beispielsweise durch bluetooth erfolgen.

Die Ansteuerung des mindestens einen Prothesenmotors erfolgt durch die Prothesensteuerungsvorrichtung 5 nach Eingabe der Parameter, insbesondere der Griffdauer und der Griffkraft. Durch den Mikrokontroller 54 wird der mindestens eine Prothesenmotor gesteuert betrieben, wobei die Parameter auch in Echtzeit während des Greifprozesses geändert werden können.

### Verfahren im Betrieb

Mit der beschriebenen Prothesensteuerungsvorrichtung 5 erfolgt hier eine Ansteuerung und die Anzeige der Griffkraft oder der Griffdauer von fremdkraftbetätigten Handprothesen 4.

Im Betrieb wird der Prothesenträger vor einer durchzuführenden Bewegung der Handprothese 4 bzw. mindestens eines Gelenkes der Handprothese 4 mittels dem mindestens einen Prothesenmotor, manuell auf der Bedienoberfläche 520 die zu erreichende Griffkraft und eventuell eine Griffdauer einstellen. Diese Einstellung geschieht mit einem Finger der intakten Hand, wobei die Parameter mit Auswahltasten 521 gewählt und die Grösse mit Grössenwahltasten 522 definiert wird. Die Anzeigemittel 53 geben dem Prothesenträger an, ob die gewünschten Einstellungen vorgenommen sind. Die Einstellungen werden von den Eingabemitteln 52 an den Mikrokontroller 54 geleitet, welcher die Ansteuerung der Ausgabemittel 53 und der Bewegung der Handprothese 4 im Folgenden durchführt.

Nach der Einstellung der gewünschten Parameter beginnt die angestrebte Bewegung, welche beispielsweise durch eine definierte Stellung der Handprothese 4 relativ zum Prothesenschaft 2 auslösbar sein kann.

Im Fall einer Greifbewegung verfolgt der Prothesenträger die Bewegung selbst, erhält aber auch eine taktile Rückkopplung, beispielsweise über Anfang und Ende der Greifbewegung mittels Vibrationen. Die Anzeigemittel 53 zeigen die Bewegungsparameter und/oder den Verlauf der Bewegung auch optisch an. Die Steuerung des mindestens einen Prothesenmotors auf Basis der vorab eingegebenen Parameter erfolgt ausgelöst und gesteuert vom Mikrokontroller 54.

Sollte der Prothesenträger erkennen, dass die Greifbewegung nicht seinen Wünschen entspricht, hat er die Möglichkeit während der Bewegung der Handprothese 4 die Parameter in Echtzeit über die Bedienoberfläche 520 zu ändern. Ebenfalls wieder manuell kann der Prothesenträger die Parameter ändern. Dabei werden dem Mikrokontroller 54 angepasste Sollwerte, beispielsweise der Griffkraft, übermittelt. Der Mikrokontroller 54 steuert dann den mindestens einen Prothesenmotor entsprechend in geänderter Form. Als Griffdauer kommt beispielsweise eine unendlich Zeitspanne oder eine konkrete Zeitspannung kleiner unendlich in Frage. Durch die Möglichkeit der nachträglichen Veränderung der Parameter, kann die Greifbewegung jederzeit beendet werden, wobei der Prothesenmotor den Griff löst und das Gelenk öffnet.

Mit der Prothesensteuerungsvorrichtung 5 kann der Prothesenträger über ein extern tragbares Modul die Griffkraft der Handprothese 4 willentlich jederzeit vorgeben. Bevor ein Objekt gegriffen wird, können Startparameter vor der Auslösung vom Prothesenträger vorgegeben werden. Sollte während der Greifbewegung erkannt werden, dass die Griffkraft zu hoch gewählt ist, da die zu greifenden Objekte weicher als gedacht sind, kann die Griffkraft einfach nachreguliert werden. So kann eine Zerstörung des gegriffenen Objektes ausgeschlossen werden.

Durch die taktile Rückkopplung mittels Vibrationsmotor 57 kann beispielsweise angezeigt werden, wann das zu greifende Objekt berührt, gegriffen und wieder losgelassen wird. Damit kann der Greifprozess dem natürlichen Greifen angenähert werden.

Die an der Prothesensteuerungsvorrichtung 5 eingestellten Parameter bzw. Rückkopplungssignale bei der Bewegung können anstelle von optischen und haptischen Signalen auch in Form von akustischer Rückkopplung übermittelt werden. Entsprechend ist mindestens ein Lautsprecher und ein Akustikmodul mit dem Mikrokontroller 54 zu verbinden, von welchem akustische Rückkoppelsignale an den Prothesenträger übermittelt werden können. Die auf dem Mikrokontroller 54 betriebene Software kann somit beispielsweise die eingestellte Griffkraft oder den erfolgreichen Abschluss einer Greifbewegung akustisch melden.

In einer weiteren Ausführungsform kann die Prothesensteuerungsvorrichtung 5 mehrteilig ausgebildet sein, wobei die Eingabemittel 52 und die Anzeigemittel 53 nicht in am Ort des Gehäuses 50 angeordnet sind, sondern örtlich voneinander getrennt sind.

Alternativ können die Eingabemittel 52 in Form des Akustikmoduls verbunden mit dem Mikrokontroller 54 ausgestaltet sein, wobei vom Akustikmodul empfangene Sprachbefehle zur Eingabe gewünschter Griffkraft und/oder Griffdauer am Mikrokontroller 54 der Prothesensteuerungsvorrichtung 5 genutzt werden. Der Prothesenträger spricht zeitlich vor der Durchführung eines Griffvorganges direkt in die Prothesensteuerungsvorrichtung 5 bzw. das Akustikmodul und gibt die Griffkraft und/oder Griffdauer durch. Anschliessend wird der verstandene Sprachbefehl durch die Anzeigemittel 53 dargestellt und der Griff kann durchgeführt werden. Damit wäre eine entsprechend sprachgesteuerte Prothese 1 erreicht.

Obwohl hier die Prothesensteuerungsvorrichtung 5 bzw. das Gehäuse 50 als Teil der Prothese 1 am Extremitätenstumpf 0 getragen wird, kann die Prothesensteuerungsvorrichtung auch an der gesunden bzw. intakten Hand getragen werden. Die Bedienung erfolgt dann beispielsweise mit einem Prothesenfinger, wenn die Eingabemittel 52 in Form der manuell bedienbaren Tasten ausgestaltet sind.

### Bezugszeichenliste

0 Extremitätenstumpf /Unterarmstumpf
1 Prothese
2 Prothesenschaft
3 Kupplung für Exoprothese
4 Exoprothese/Handprothese
5 Prothesensteuerungsvorrichtung
50 Gehäuse
51 Armband
52 Eingabemittel
520 Bedienoberfläche
521 Auswahltasten
522 Grössenwahltasten
53 Anzeigemittel (Touchdisplay, LEDs)
53' LEDs
54 Mikrokontroller
55 Spannungsversorgung
56 haptischer Motorentreiber
57 Vibrationsmotor
58 Beschleunigungssensor

## Patentansprüche

1. Fremdkraftbetätigbare Prothese (1), umfassend einen Prothesenschaft (2) und eine Exoprothese (4) mit mindestens einem mittels einer Prothesensteuerungsvorrichtung (5) betätigbaren Prothesenmotor und einem Gelenk,
wobei die Prothesensteuerungsvorrichtung (5) ein Gehäuse (50), einen darin angeordneten Mikrokontroller (54) und Anzeigemittel (53) umfasst, sodass ein Greifprozess durch den von der Prothesensteuerungsvorrichtung (5) steuerbaren Prothesenmotor nachbildbar ist und Parameter des Greifprozesses von den Anzeigemitteln (53) darstellbar sind, wobei auf die Aufnahme und Auswertung von Elektromyografie-Signalen verzichtet wird, wobei die Anzeigemittel (53) gewählte Parameter eines kommenden Greifprozesses anzeigen und die Prothesensteuerungsvorrichtung (5) Eingabemittel (52) umfasst, mittels welchen eine gewünschte Griffkraft und/oder Griffdauer am Mikrokontroller (54) der Prothesensteuerungsvorrichtung (5) zeitlich vor der Durchführung des Greifprozesses durch einen Prothesenträger direkt an der Prothesensteuerungsvorrichtung (5) aktiv einstellbar ist und während des Greifprozesses mittels der Eingabemittel (52) nachregelbar ist, **dadurch gekennzeichnet, dass** mindestens ein Akustikmodul und ein Lautsprecher mit dem Mikrokontroller (54) der Prothesensteuerungsvorrichtung (5) verbunden sind, welche eine akustische Rückkopplung der eingestellten Parameter des Greifprozesses vor und während des Greifprozesses an den Prothesenträger übermitteln.

2. Fremdkraftbetätigbare Prothese (1) nach Anspruch 1, wobei die Eingabemittel (52) in Form von Auswahltasten (521) und Grössenwahltasten (522) auf einer Bedienoberfläche (520) gewählt sind, mittels welchen die Einstellungen an der Prothesensteuerungsvorrichtung (5) zeitlich vor der Durchführung des Greifprozesses durch einen Prothesenträger direkt an der Prothesensteuerungsvorrichtung (5) erfolgen.

3. Fremdkraftbetätigbare Prothese (1) nach Anspruch 2, wobei die Eingabemittel (52) auf der Bedienoberfläche (520) als kapazitive oder resistive Schalter (52) oder Druckelemente (52) ausgebildet sind, welche vom Prothesenträger manuell mechanisch betätigbar sind.

4. Fremdkraftbetätigbare Prothese (1) nach Anspruch 2, wobei die Bedienoberfläche (520) in Form eines berührungsempfindlichen Bildschirms ausgestaltet ist und die Eingabemittel (52) auf der Bedienoberfläche (520) als virtuelle Eingabeknöpfen gewählt sind.

5. Fremdkraftbetätigbare Prothese (1) nach einem der Ansprüche 2 bis 4, wobei die Bedienoberfläche (520) als berührungsempfindliches PCB-Bauteil in Form einer Leiterplatte ausgebildet ist, wobei die Auswahltasten (521) und die Grössenwahltasten (522) berührungsempfindliche Flächen (touch-pads) sind.

6. Fremdkraftbetätigbare Prothese (1) nach einem der Ansprüche 2 bis 5, wobei die Anzeigemittel (53) als Darstellungen auf einem berührungsempfindlichen Bildschirm ausgestaltet sind und die Auswahltasten (521) und die Grössenwahltasten (522) auf dem berührungsempfindlichen Bildschirm eingebbar und ablesbar sind.

7. Fremdkraftbetätigbare Prothese (1) nach einem der Ansprüche 1 bis 5, wobei die Anzeigemittel (53) LEDs (53') sind, wobei die Anzahl leuchtender LEDs (53') oder die Farbe der leuchtenden LEDs (53') die gewählten Parameter optisch anzeigen.

8. Fremdkraftbetätigbare Prothese (1) nach einem der vorhergehenden Ansprüche, wobei ein Vibrationsmotor (57) mit einem haptischen Motorentreiber (56) mit dem Mikrokontroller (54) verbunden ist, sodass eingestellte Parameter bzw. Rückkoppelsignale des Greifprozesses haptisch an den Prothesenträger übermittelt werden.

9. Fremdkraftbetätigbare Prothese (1) nach einem der vorhergehenden Ansprüche, wobei die Eingabemittel (52) in Form des Akustikmoduls verbunden mit dem Mikrokontroller (54) ausgebildet sind, sodass Sprachbefehle zur Eingabe gewünschter Griffkraft und/oder Griffdauer am Mikrokontroller (54) der Prothesensteuerungsvorrichtung (5) zeitlich vor der Durchführung des Greifprozesses durch einen Prothesenträger direkt an der Prothesensteuerungsvorrichtung (5) nutzbar sind.

10. Fremdkraftbetätigbare Prothese (1) nach einem der vorhergehenden Ansprüche, wobei der Mikrokontroller (54) auf einem Einplatinencomputer angeordnet ist und eine Betriebssoftware zur Einstellung und Anzeige der Eingabeparameter und zur Steuerung des Greifprozesses und mindestens eine Applikation zur Musik- oder Videowiedergabe, als Spieleanwendung, zur Nachrichtenübermittlung oder zum Telefonieren.

11. Fremdkraftbetätigbare Prothese (1) nach einem der vorhergehenden Ansprüche, wobei das Gehäuse (50) samt Mikrokontroller (54), Eingabemittel (52), Anzeigemittel (53) in die Exoprothese (4) integriert angeordnet ist.

12. Fremdkraftbetätigbare Prothese (1) nach einem der Ansprüche 1 bis 10, wobei das Gehäuse (50) in Form eines Armbandes (51) ausgestaltet ist und um den Prothesenschaft (2) oder eine Kupplung (3) zwischen Prothesenschaft (2) und Exoprothese (4) gelegt ist.

13. Fremdkraftbetätigbare Prothese (1) nach einem der vorhergehenden Ansprüche, wobei die Verbindung zwischen Prothesensteuerungsvorrichtung (5) und dem mindestens einen Prothesenmotor drahtlos, bevorzugt durch bluetooth ausgestaltet ist.

## Claims

1. Prosthesis (1) actuable by external force, comprising a prosthesis shaft (2) and an exoprosthesis (4) with at least one prosthesis motor actuable by means of a prosthesis control device (5) and a joint,
wherein the prosthesis control device (5) comprises a housing (50), a microcontroller (54) arranged therein and display means (53), so that a gripping process can be reproduced by the prosthesis motor controllable by the prosthesis control device (5) and parameters of the gripping process can be displayed by the display means (53), wherein the recording and evaluation of electromyography signals is dispensed with, wherein the display means (53) display selected parameters of an upcoming gripping process and the prosthesis control device (5) comprises input means (52), by means of which a desired gripping force and/or gripping duration can be actively set at the microcontroller (54) of the prosthesis control device (5) directly on the prosthesis control device (5) temporally before the gripping process is carried out by a prosthesis wearer and can be readjusted during the gripping process by means of the input means (52), **characterised in that** at least one acoustic module and a loudspeaker are connected to the microcontroller (54) of the prosthesis control device (5), which transmit acoustic feedback of the set parameters of the gripping process to the prosthesis wearer before and during the gripping process.

2. Prosthesis (1) actuable by external force according to claim 1, wherein the input means (52) are selected in the form of selection keys (521) and size selection keys (522) on a user interface (520), by means of which the settings on the prosthesis control device (5) are made directly on the prosthesis control device (5) temporally before the gripping process is carried out by a prosthesis wearer.

3. Prosthesis (1) actuable by external force according to claim 2, wherein the input means (52) on the user interface (520) are embodied as capacitive or resistive switches (52) or pressure elements (52), which can be manually mechanically actuated by the prosthesis wearer.

4. Prosthesis (1) actuable by external force according to claim 2, wherein the user interface (520) is embodied in the form of a touch-sensitive screen and the input means (52) on the user interface (520) are selected as virtual input buttons.

5. Prosthesis (1) actuable by external force according to any one of claims 2 to 4, wherein the user interface (520) is embodied as a touch-sensitive PCB component in the form of a printed circuit board, wherein the selection keys (521) and the size selection keys (522) are touch-sensitive surfaces.

6. Prosthesis (1) actuable by external force according to any one of the claims 2 to 5, wherein the display means (53) are embodied as representations on a touch-sensitive screen and the selection keys (521) and the size selection keys (522) can be entered and read on the touch-sensitive screen.

7. Prosthesis (1) actuable by external force according to any one of claims 1 to 5, wherein the display means (53) are LEDs (53'), the number of illuminated LEDs (53') or the colour of the illuminated LEDs (53') visually indicating the selected parameters.

8. Prosthesis (1) actuable by external force according to any one of the preceding claims, wherein a vibration motor (57) with a haptic motor driver (56) is connected to the microcontroller (54) so that set parameters or feedback signals of the gripping process are haptically transmitted to the prosthesis wearer.

9. Prosthesis (1) actuable by external force according to any one of the preceding claims, wherein the input means (52) in the form of the acoustic module are connected to the microcontroller (54) so that voice commands for inputting desired gripping force and/or gripping duration at the microcontroller (54) of the prosthesis control device (5) temporally before the gripping process is carried out by a prosthesis wearer directly at the prosthesis control device (5) can be used.

10. Prosthesis (1) actuable by external force according to any one of the preceding claims, wherein the microcontroller (54) is arranged on a single board computer and an operating software for setting and displaying the input parameters and for controlling the gripping process and at least one application for music or video playback, as a game application, for messaging or for telephoning, is used.

11. Prosthesis (1) actuable by external force according to any one of the preceding claims, wherein the housing (50) together with microcontroller (54), input means (52), display means (53) is arranged integrated in the exoprosthesis (4).

12. Prosthesis (1) actuable by external force according to any one of claims 1 to 10, wherein the housing (50) is embodied in the form of a bracelet (51) and is placed around the prosthesis shaft (2) or a coupling (3) between prosthesis shaft (2) and exoprosthesis (4).

13. Prosthesis (1) actuable by external force according to any one of the preceding claims, wherein the connection between the prosthesis control device (5) and the at least one prosthesis motor is wireless, preferably bluetooth.

## Revendications

1. Prothèse (1) configurée de manière à pouvoir être actionnée par une force extérieure, comprenant une tige de prothèse (2) et une exoprothèse (4) avec au moins un moteur de prothèse configuré de manière à pouvoir être actionné au moyen d'un dispositif de commande de prothèse (5) et une articulation, dans laquelle le dispositif de commande de prothèse (5) comprend un boîtier (50), un microcontrôleur (54) disposé dans celui-ci et des moyens d'affichage (53), de sorte qu'un processus de préhension est reproductible par le moteur de prothèse configuré de manière à pouvoir être commandé par le dispositif de commande de prothèse (5) et des paramètres du processus de préhension sont configurés de manière à pouvoir être représentés par les moyens d'affichage (53), dans laquelle il est renoncé à l'enregistrement et à l'évaluation de signaux d'électromyographie, dans laquelle les moyens d'affichage (53) affichent des paramètres sélectionnés d'un processus de préhension à venir et le dispositif de commande de prothèse (5) comprend des moyens de saisie (52), au moyen desquels une force de préhension souhaitée et/ou une durée de préhension souhaitée au niveau du microcontrôleur (54) du dispositif de commande de prothèse (5) est réglable temporellement de manière active avant l'exécution du processus de préhension par un porteur de prothèse directement au niveau du dispositif de commande de prothèse (5) et est réajustable pendant le processus de préhension au moyen des moyens de saisie (52), **caractérisée en ce qu'**au moins un module acoustique et un haut-parleur sont reliés au microcontrôleur (54) du dispositif de commande de prothèse (5), lesquels transmettent au porteur de prothèse une rétroaction acoustique des paramètres réglés du processus de préhension avant et pendant le processus de préhension.

2. Prothèse (1) configurée de manière à pouvoir être actionnée par une force extérieure selon la revendication 1, dans laquelle les moyens de saisie (52) sont sélectionnés sous la forme de touches de sélection (521) et de touches de sélection de taille (522) sur une interface de commande (520), au moyen desquelles les réglages au niveau du dispositif de commande de prothèse (5) sont effectués temporellement avant l'exécution du processus de préhension par un porteur de prothèse directement au niveau du dispositif de commande de prothèse (5).

3. Prothèse (1) configurée de manière à pouvoir être actionnée par une force extérieure selon la revendication 2, dans laquelle les moyens de saisie (52) sont configurés sur l'interface de commande (520) sous la forme de commutateurs capacitifs ou résistifs (52) ou d'éléments de pression (52), lesquels sont configurés de manière à pouvoir être actionnés mécaniquement manuellement par le porteur de prothèse.

4. Prothèse (1) configurée de manière à pouvoir être actionnée par une force extérieure selon la revendication 2, dans laquelle l'interface de commande (520) est configurée sous la forme d'un écran tactile et les moyens de saisie (52) sont sélectionnés sur l'interface de commande (520) sous la forme de boutons de saisie virtuels.

5. Prothèse (1) configurée de manière à pouvoir être actionnée par une force extérieure selon l'une des revendications 2 à 4, dans laquelle l'interface de commande (520) est configurée sous la forme d'un composant PCB tactile sous la forme d'une carte de circuit imprimé, dans laquelle les touches de sélection (521) et les touches de sélection de taille (522) sont des surfaces tactiles (pads tactiles).

6. Prothèse (1) configurée de manière à pouvoir être actionnée par une force extérieure selon l'une des revendications 2 à 5, dans laquelle les moyens d'affichage (53) sont configurés sous la forme de représentations sur un écran tactile et les touches de sélection (521) ainsi que les touches de sélection de taille (522) sont configurées de manière à pouvoir être saisies et lues sur l'écran tactile.

7. Prothèse (1) configurée de manière à pouvoir être actionnée par une force extérieure selon l'une des revendications 1 à 5, dans laquelle les moyens d'affichage (53) sont des LED (53'), dans laquelle le nombre de LED lumineuses (53') ou la couleur des LED lumineuses (53') indique visuellement les paramètres sélectionnés.

8. Prothèse (1) configurée de manière à pouvoir être actionnée par une force extérieure selon l'une des revendications précédentes, dans laquelle un moteur vibrant (57) avec un pilote de moteur haptique (56) est relié au microcontrôleur (54), de sorte que des paramètres réglés ou des signaux de rétroaction du processus de préhension sont transmis de manière haptique au porteur de prothèse.

9. Prothèse (1) configurée de manière à pouvoir être actionnée par une force extérieure selon l'une des revendications précédentes, dans laquelle les moyens de saisie (52) sont configurés sous la forme du module acoustique relié au microcontrôleur (54), de sorte que des commandes vocales pour la saisie d'une force de préhension souhaitée et/ou d'une durée de préhension souhaitée au niveau du microcontrôleur (54) du dispositif de commande de prothèse (5) sont utilisables temporellement avant l'exécution du processus de préhension par un porteur de prothèse directement au niveau du dispositif de commande de prothèse (5).

10. Prothèse (1) configurée de manière à pouvoir être actionnée par une force extérieure selon l'une des revendications précédentes, dans laquelle le microcontrôleur (54) est disposé sur un ordinateur monocarte et comprend un logiciel d'exploitation pour le réglage et l'affichage des paramètres de saisie et pour la commande du processus de préhension et au moins une application pour la reproduction de musique ou de vidéo, en tant qu'application de jeu, pour la transmission de messages ou pour la téléphonie.

11. Prothèse (1) configurée de manière à pouvoir être actionnée par une force extérieure selon l'une des revendications précédentes, dans laquelle le boîtier (50) avec le microcontrôleur (54), les moyens de saisie (52), les moyens d'affichage (53) est disposé de manière intégrée dans l'exoprothèse (4).

12. Prothèse (1) configurée de manière à pouvoir être actionnée par une force extérieure selon l'une des revendications 1 à 10, dans laquelle le boîtier (50) est configuré sous la forme d'un bracelet (51) et est posé autour de la tige de prothèse (2) ou d'un raccord (3) entre la tige de prothèse (2) et l'exoprothèse (4).

13. Prothèse (1) configurée de manière à pouvoir être actionnée par une force extérieure selon l'une des revendications précédentes, dans laquelle la liaison entre le dispositif de commande de prothèse (5) et l'au moins un moteur de prothèse est configurée sans fil, de préférence par Bluetooth.
